# EUROPEAN PATENT SPECIFICATION

(11) **EP 3 238 699 B1**
(45) Date of publication and mention of the grant of the patent: **25.03.2020**
(21) Application number: 15873263.6
(22) Date of filing: 25.12.2015
(51) Int. Cl.: A61K 8/44, A61Q 19/00, A61K 8/81, A61Q 5/00, A61Q 9/02, A61Q 17/04, C09K 3/00

(54) **COSMETIC COMPOSITION CONTAINING ACYL BASIC AMINO ACID DERIVATIVE AND ANIONIC WATER-SOLUBLE POLYMER**
KOSMETISCHE ZUSAMMENSETZUNG MIT ACYLBASISCHEM AMINOSÄUREDERIVAT UND IN ANIONISCHEM WASSER LÖSLICHEM POLYMER
COMPOSITION COSMÉTIQUE CONTENANT UN DÉRIVÉ D'ACIDE AMINÉ DE BASE ACYLE ET UN POLYMÈRE SOLUBLE DANS L'EAU ANIONIQUE

(30) Priority: 25.12.2014 JP 2014262412
(43) Date of publication of application: 01.11.2017
(73) Proprietor: AJINOMOTO CO., INC., Chuo-ku Tokyo 104-8315 (JP)
(72) Inventor: HARAYA, Nana, Kawasaki-shi Kanagawa 210-8681 (JP); KOBAYASHI, Shun, Kawasaki-shi Kanagawa 210-8681 (JP); KURAMOTO, Masayuki, Yokkaichi-shi Mie 510-0885 (JP)
(74) Representative: Mewburn Ellis LLP
(86) International application number: PCT/JP2015/086212
(87) International publication number: WO 2016/104695

(56) References cited:
- EP-A1- 1 473 027
- EP-A1- 2 813 487
- WO-A1-2013/118896
- JP-A- 2004 323 505
- MASAHIRO SUZUKI ET AL.: 'Novel dumbbell-form low- molecular-weight gelators based on L-lysine: their hydrogelation and organogelation properties' NEW JOURNAL OF CHEMISTRY vol. 29, no. 11, 01 January 2005, pages 1439 - 1444, XP055346030 DOI: 10.1039/B511158G

## Description

### [Technical Field]

The present invention relates to a composition containing (A): an acyl basic amino acid derivative and (B): an anionic water-soluble polymer, which is used as, for example, cosmetics.

### [Background Art]

Thickeners such as anionic water-soluble polymer and the like are used for cosmetics to enhance usability, stability and the like and to prevent dripping. For example, since acrylic water-soluble polymers such as carboxyvinyl polymer and the like have high thickening property, cause less stimulation, are superior in transparency, and afford good sense of use with less stickiness, they are widely used for thickening the base of a wide range of compositions for skincare, haircare and the like (see, e.g. EP2813487).

However, since acrylic water-soluble polymers are thickened by neutralization, the thickening property is shown in a limited pH range, and expected viscosity may not be afforded at a desired pH. In addition, acrylic water-soluble polymers are known to show a marked decrease in the viscosity when they are co-present with electrolytes such as salt and the like (non-patent document 1). Furthermore, when a large amount of alcohol is added to an acrylic water-soluble polymer, the hydration of the acrylic water-soluble polymer becomes unstable, which in turn often causes aggregation and decrease in the viscosity.

When an acrylic water-soluble polymer having a high concentration is added to compensate for such decrease in the viscosity, stickiness and sliminess are produced at the time of application, and there is an essential disadvantage that sense of use is impaired (patent document 1).

It has been reported that a compound represented by the following formula: 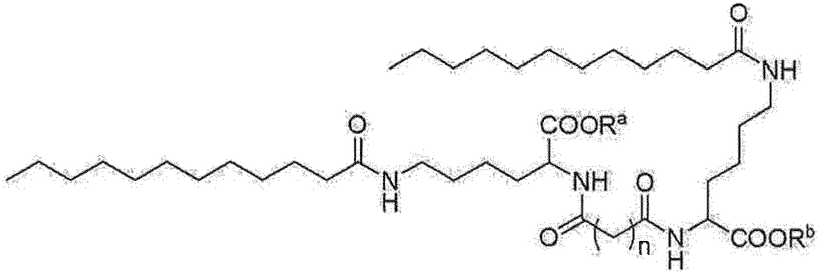 wherein R^{a} and R^{b} are each a hydrogen atom or an alkyl group, and n is an integer of 0 to 12, or a salt thereof (hereinafter to be also referred to as "lauroyl amino acid derivative") is useful for gelation or solidifying water and a liquid organic medium (patent documents 2 and 3, non-patent document 2 and non-patent document 3 etc.). The lauroyl amino acid derivative blended in cosmetics is expected to improve affinity to the skin, as well as exhibit moisturizing ability. Therefore, incorporation of lauroyl amino acid derivatives in cosmetics has been studied.

However, when a lauroyl amino acid derivative is added to liquid cosmetics such as skin lotion, milky lotion and the like, usability is problematically limited since water is separated (syneresis) with time even though gel may be formed once, aggregates are non-uniformly formed in an acidic range, and the like, .

### [Document List]

### [Patent documents]

patent document 1: JP-A-2005-120056
patent document 2: JP-A-2004-323505 patent document 3: EP1473027

### [non-patent document]

non-patent document 1: water-soluble polymer technique and market of environment-adaptable polymers, CMC Publishing Co., Ltd. (1992), 124-127
non-patent document 2: Org. Biomol. Chem., 2003, 1, 4124-4131 non-patent document 3: New J. Chem., 2005, 29, 1439-1444

### [SUMMARY OF THE INVENTION]

### [Problems to be Solved by the Invention]

An object of the present invention is to provide a composition, which suppresses syneresis and aggregation over time caused by a lauroyl amino acid derivative, maintains desired viscosity in a wide pH range even in the presence of electrolyte or alcohol, and shows good usability by suppressing stickiness and sliminess.

### [Means of Solving the Problems]

The present inventors have conducted intensive studies in an attempt to achieve the above-mentioned object, and found that syneresis and aggregation over time do not occur, desired viscosity is maintained in a wide pH range even in the presence of electrolyte or alcohol, and stickiness and sliminess relating to usability can be suppressed by merely adding component (B): an anionic water-soluble polymer to component (A) which is a lauroyl amino acid derivative: a compound represented by the following formula (1) (hereinafter to be also referred to as "compound (1)") or a salt thereof, which resulted in the completion of the present invention.

Therefore, the present invention provides the following.
[1] A composition comprising component (A): a compound represented by the formula (1) 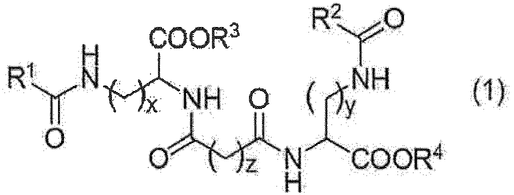 wherein
   R¹ and R² are each independently an alkyl group having 5 - 21 carbon atoms or an alkenyl group having 5 - 21 carbon atoms,
   R³ and R⁴ are each independently a hydrogen atom, an alkyl group having 1 - 22 carbon atoms or an alkenyl group having 2 - 22 carbon atoms, z is 7 or 8,
   x and y are each independently an integer of 2 - 4, or a salt thereof, and component (B): an anionic water-soluble polymer.
[2] The composition of [1], wherein component (A) is a compound of the aforementioned formula (1) wherein x and y are each 4, or a salt thereof.
[3] The composition of [1] or [2], wherein component (A) is a compound of the aforementioned formula (1) wherein R¹ and R² are each independently a straight-chain alkyl group having 5 - 15 carbon atoms, or a salt thereof.
[4] The composition of any of [1] - [3], wherein component (A) is a compound of the aforementioned formula (1) wherein R³ and R⁴ are each a hydrogen atom, or a salt thereof.
[5] The composition of any of [1] - [4] wherein component (A) is a compound of the aforementioned formula (1) wherein R¹ and R² are each a straight-chain alkyl group having 5 - 15 carbon atoms, R³ and R⁴ are each a hydrogen atom, z is 7 or 8, and x and y are each 4, or a salt thereof.
[6] The composition of any of [1] - [5], wherein component (A) is a compound selected from bis(N^{ε}-lauroyl-L-lysine)sebacoyl amide and bis(N^{ε}-octanoyl-L-lysine)sebacoyl amide, or a salt thereof.
[7] The composition of any of [1] - [6], wherein component (B) is an anionic water-soluble polymer having a carboxyl group.
[8] The composition of any of [1] - [6], wherein component (B) is at least one kind of anionic water-soluble polymer selected from the group consisting of a carboxyvinyl polymer, polyacrylic acid or a salt thereof, crosslinked polyacrylic acid or a salt thereof, (acrylic acid/(C10-30)alkyl acrylate) copolymer, alkyl acrylate copolymer fluid, carboxymethylcellulose or a salt thereof, xanthan gum, and alginic acid or a salt thereof.
[9] The composition of any of [1] - [8], further comprising component (C): electrolyte or component (D):alcohol.
[10] The composition of any of [1] - [9], wherein component (A) is contained in a proportion of 0.001 - 10 wt%, relative to the total amount of the composition.
[11] The composition of any of [1] - [10], wherein the component (B) is contained in a proportion of 0.05 - 10 wt% relative to the total amount of the composition.
[12] The composition of any of [1] - [11], wherein the weight of component (A)/weight of component (B) is 1/99 - 99/1.
[13] The composition of any of [9] - [12], wherein component (C) is at least one kind selected from the group consisting of sodium chloride, sodium pyrrolidone carboxylate and sodium lactate.
[14] The composition of any of [9] - [12], wherein component (D) is at least one kind of alcohol selected from the group consisting of ethanol, propanol and isopropanol.
[15] A cosmetic comprising the composition of any of [1] - [14].
[16] A method of producing a composition with improved viscosity stability, comprising a step of blending component (A): a compound represented by the formula (1) 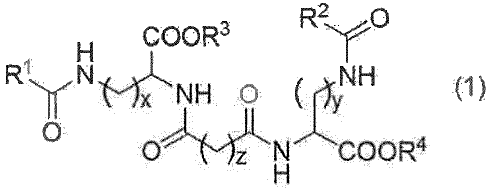 wherein each symbol is as defined in [1], or a salt thereof, and component (B): an anionic water-soluble polymer.
[17] A method of suppressing syneresis or aggregation of a composition, comprising a step of adding component (B): an anionic water-soluble polymer to component (A): compound (1) or a salt thereof.
[18] A method of producing a composition with improved viscosity stability, comprising a step of adding component (A): compound (1) or a salt thereof to component (B): an anionic water-soluble polymer in the presence of electrolyte or alcohol.
[19] A method of stabilizing viscosity of a composition, comprising a step of adding component (A): compound (1) or a salt thereof to component (B): an anionic water-soluble polymer in the presence of electrolyte or alcohol.

### [Effect of the Invention]

According to the present invention, a composition which suppresses syneresis and aggregation over time, maintains desired viscosity in a wide pH range even in the presence of electrolyte or alcohol, and shows good usability by suppressing stickiness and sliminess can be provided.

### [Description of Embodiments]

The composition of the present invention is characterized in that it is a composition containing component (A): a compound represented by the formula (1) 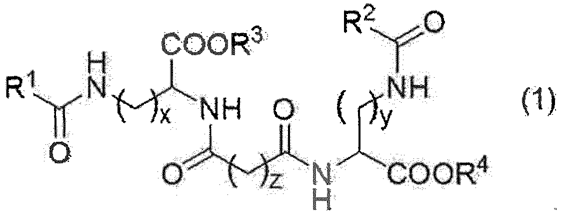 wherein
R¹ and R² are each independently an alkyl group having 5 - 21 carbon atoms or an alkenyl group having 5 - 21 carbon atoms,
R³ and R⁴ are each independently a hydrogen atom, an alkyl group having 1 - 22 carbon atoms or an alkenyl group having 2 - 22 carbon atoms, z is 7 or 8,
x and y are each independently an integer of 2 - 4, or a salt thereof, and
component (B): an anionic water-soluble polymer.

In addition, the composition of the present invention is characterized in that it is a composition further containing component (C): electrolyte or component (D): alcohol in addition to component (A) and component (B).

The embodiment of the present invention is described in detail in the following.

### 1. Component (A): a compound represented by the formula (1) (compound (1)) or a salt thereof

R¹ and R² are each independently an alkyl group having 5 - 21 carbon atoms or an alkenyl group having 5 - 21 carbon atoms.

The alkyl group having 5 - 21 carbon atoms means a straight-chain or branched-chain alkyl group having 5 - 21 carbon atoms. Specific examples thereof include pentyl group, isopentyl group, neopentyl group, a hexyl group, isohexyl group, neohexyl group, heptyl group, isoheptyl group, neoheptyl group, octyl group, isooctyl group, nonyl group, isononyl group, decyl group, isodecyl group, undecyl group, dodecyl group, tridecyl group, tetradecyl group, pentadecyl group, hexadecyl group, heptadecyl group, octadecyl group, nonadecyl group, icosyl group and the like.

The alkenyl group having 5 - 21 carbon atoms means a straight-chain or branched-chain alkenyl group having 5 - 21 carbon atoms. Specific examples thereof include pentenyl group, hexenyl group, heptenyl group, octenyl group, nonenyl group, decenyl group, undecenyl group, dodecenyl group, tridecenyl group, tetradecenyl group, pentadecenyl group, hexadecenyl group, heptadecenyl group, octadecenyl group, nonadecenyl group, icosenyl group and the like.

An alkyl group having 5 - 15 carbon atoms means a straight-chain or branched-chain alkyl group having 5 - 15 carbon atoms. Specific examples thereof include pentyl group, a hexyl group, heptyl group, octyl group, nonyl group, decyl group, undecyl group, dodecyl group, tridecyl group, tetradecyl group, pentadecyl group and the like.

An alkyl group having 7 - 11 carbon atoms means a straight-chain or branched-chain alkyl group having 7 - 11 carbon atoms. Specific examples thereof include heptyl group, octyl group, nonyl group, decyl group, undecyl group and the like.

R¹ and R² are preferably each independently an alkyl group having 5 - 15 carbon atoms, more preferably each independently an alkyl group having 7 - 11 carbon atoms.

Preferably, R¹ and R² are each a straight chain alkyl group. Furthermore, R¹ and R² are preferably the same.

R³ and R⁴ are each independently a hydrogen atom, an alkyl group having 1 - 22 carbon atoms or an alkenyl group having 2 - 22 carbon atoms.

An alkyl group having 1 - 22 carbon atoms means a straight-chain or branched-chain alkyl group having 1 - 22 carbon atoms. Specific examples thereof include methyl group, an ethyl group, a propyl group, an isopropyl group, a butyl group, an isobutyl group, a sec-butyl group, a tert-butyl group, a pentyl group, isopentyl group, neopentyl group, a hexyl group, isohexyl group, neohexyl group, heptyl group, isoheptyl group, neoheptyl group, octyl group, isooctyl group, nonyl group, isononyl group, decyl group, isodecyl group, undecyl group, dodecyl group, tridecyl group, tetradecyl group, pentadecyl group, hexadecyl group, heptadecyl group, octadecyl group, nonadecyl group, icosyl group and the like.

An alkenyl group having 2 - 22 carbon atoms means a straight-chain or branched-chain alkenyl group having 2 - 22 carbon atoms. Specific examples thereof include ethenyl group, 1-propenyl group, 2-propenyl group, 1-butenyl group, 2-butenyl group, 3-butenyl group, pentenyl group, hexenyl group, heptenyl group, octenyl group, nonenyl group, decenyl group, undecenyl group, dodecenyl group, tridecenyl group, tetradecenyl group, pentadecenyl group, hexadecenyl group, heptadecenyl group, octadecenyl group, nonadecenyl group, icosenyl group and the like.

Preferably, both R³ and R⁴ are hydrogen atoms.

z is 7 or 8.

x and y are each independently an integer of 2 - 4.
x and y are each preferably 4.

As a compound represented by the formula (1), the following compounds can be preferably recited.

### (compound A)

A compound wherein R¹ and R² are each a straight chain alkyl group having 5 - 15 carbon atoms,
R³ and R⁴ are each a hydrogen atom,
z is 7 or 8, and
x and y are each 4.

### (compound C)

A compound wherein R¹ and R² are each a straight chain alkyl group having 7 - 11 carbon atoms,
R³ and R⁴ are each a hydrogen atom,
z is 7 or 8, and
x and y are each 4.

Specific examples of the compound represented by the formula (1) include
bis(N^{ε}-lauroyl-L-lysine)sebacoyl amide,
bis(N^{ε}-octanoyl-L-lysine)sebacoyl amide, and a salt thereof.

The salt of the compound represented by the formula (1) is not particularly limited. Examples thereof include alkali metal salts such as sodium salt, potassium salt and the like, alkaline earth metal salts such as calcium salt, magnesium salt and the like, inorganic salts such as aluminum salt, salt with zinc and the like, and organic salts such as organic amine salts such as ammonium salt, monoethanolamine salt, diethanolamine salt, triethanolamine salt and the like, basic amino acid salts such as arginine salt, lysine salt and the like, and the like. One kind of these may be used, or two or more kinds selected from the above-mentioned group may be used in a mixture. From the aspects of easy availability, handling property and the like, alkali metal salt, organic amine salt, or basic amino acid salt is preferable, and sodium salt and potassium salt are particularly preferable.

Compound (1) can be produced by a method conventional method (JP-A-2004-323505, Org. Biomol. Chem., 2003, 1, 4124-4131, New J. Chem., 2005, 29, 1439-1444 etc.). For example, as shown in the following formula, of compounds (1), symmetrical compound (1') can be produced by reacting N^{ω}-acyl amino acid (2) and dicarboxylic acid dichloride (3) in an appropriate solvent. 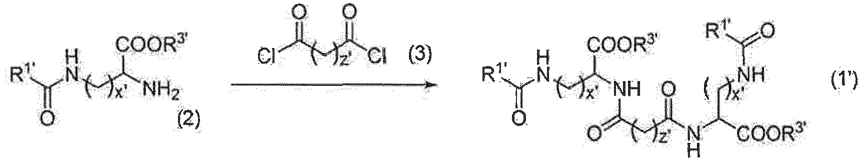 wherein R^{1'} is an alkyl group having 5 - 21 carbon atoms or an alkenyl group having 5 - 21 carbon atoms, R^{3'} is a hydrogen atom, an alkyl group having 1 - 22 carbon atoms or an alkenyl group having 2 - 22 carbon atoms, z' is an integer of not less than 0, and x' is an integer of 2 - 4.

Examples of the N^{ω}-acyl amino acid (2) include N^{ε}-acyl lysine (e.g., N^{ε}-hexanoyl-L-lysine, N^{ε}-octanoyl-L-lysine etc.), N^{δ}-acyl ornithine (e.g., N^{δ}-hexanoyl-L-ornithine etc.), N^{γ}-acyl-α,γ-diaminobutyric acid and the like.

Examples of the dicarboxylic acid dichloride (3) include azelaoyl chloride and sebacoyl chloride. The amount of dicarboxylic acid dichloride (3) to be used is generally 0.4 - 0.6 equivalent relative to N^{ω}-acyl amino acid (2).

While the solvent is not particularly limited as long as it is inert to the reaction, examples thereof include ethers such as diethyl ether, tetrahydrofuran and the like.

In addition, of compounds (1), asymmetric compound (1") can be produced as follows. First, N^{ω}-acyl amino acid (2) and dicarboxylic acid monochloride monoester (4) are reacted in an appropriate solvent to give compound (5) (step 1). Then, the primary ester moiety of the obtained compound (5) is hydrolyzed in the presence of a base such as sodium hydroxide, potassium hydroxide and the like, the carboxylic acid moiety is chlorinated with a chlorinating agent such as thionyl chloride and the like, and the compound is reacted with N^{ω}-acyl amino acid (2') which is different from N^{ω}-acyl amino acid (2) used in the aforementioned step 1 (step 2), whereby derivative (1") can be produced. 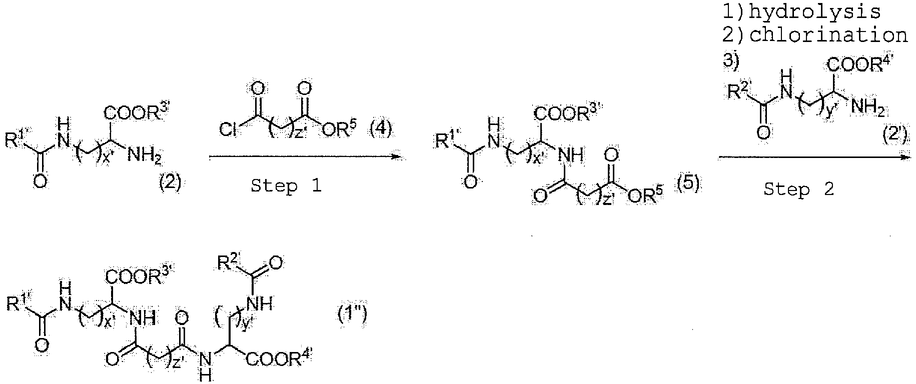 wherein R^{1'}, R^{3'}, z' and x' are as defined above, R^{2'} is an alkyl group having 5 - 21 carbon atoms or an alkenyl group having 5 - 21 carbon atoms, R^{4'} is a hydrogen atom, an alkyl group having 1 - 22 carbon atoms or an alkenyl group having 2 - 22 carbon atoms, R⁵ is an alkyl group such as a methyl group, an ethyl group and the like, and y' is an integer of 2 - 4.

As N^{ω}-acyl amino acids (2) and (2'), N^{ω}-acyl amino acids similar to those mentioned above can be used.

As dicarboxylic acid monochloride monoester (4), a commercially available product can be used as is when it is commercially available, or one produced by a method known per se or a method analogous thereto can also be used.

The acyl basic amino acid derivative obtained by the aforementioned method can be converted to a salt of acyl basic amino acid derivative by a reaction with alkali metal hydroxide such as sodium hydroxide, potassium hydroxide and the like, alkali earth metal hydroxide such as calcium hydroxide and the like, organic amine base, or the like.

The content of component (A): acyl basic amino acid derivative or a salt thereof in the composition of the present invention is generally 0.001 - 10 wt%, preferably 0.005 - 5 wt%, relative to the total amount of the composition.

### 2. Component (B): anionic water-soluble polymer

In the present specification, the "anionic water-soluble polymer" means a negatively-charged water-soluble polymer. While the "anionic water-soluble polymer" is not particularly limited as long as it is a negatively-charged water-soluble polymer, it preferably has a carboxyl group in a molecule from the aspect of usability. Specific examples of the "anionic water-soluble polymer" include carboxyvinyl polymer, polyacrylic acid or a salt thereof, crosslinked polyacrylic acid or a salt thereof, (acrylic acid/(C10-30)alkyl acrylate) copolymer, alkyl acrylate copolymer fluid, (acrylates/alkyl acrylate) crosspolymer, (hydroxyethyl acrylate/sodium acryloyl dimethyl taurine) copolymer, carboxymethylcellulose or a salt thereof, xanthan gum, and alginic acid or a salt thereof and the like. Preferred are carboxyvinyl polymer, polyacrylic acid or a salt thereof, crosslinked polyacrylic acid or a salt thereof, (acrylic acid/(C10-30) alkyl acrylate) copolymer, (acrylates/alkyl acrylate) crosspolymer and the like.

Examples of the salt of the anionic water-soluble polymer include salts similar to those exemplified as the salt of the aforementioned compound represented by the formula (1).

The anionic water-soluble polymer may be used alone or two or more kinds thereof may be used in a mixture.

The content of component (B): an anionic water-soluble polymer in the composition of the present invention is generally 0.05 - 10 wt%, preferably 0.05 - 5 wt%, relative to the total amount of the composition.

The weight of component (A)/weight of component (B) in the composition of the present invention is generally 1/99 - 99/1, preferably 1/4 - 4/1.

The form of the composition of the present invention is generally viscous liquid, gel, cream, stick and the like, preferably viscous liquid, gel, cream and the like.

### 3. Component (C): electrolyte

In the present specification, the "electrolyte" means a substance that dissociates into cation and anion when dissolved in a solvent, and shows conductivity. Specific examples of the "electrolyte" include sodium chloride, potassium chloride, magnesium chloride, calcium chloride, magnesium sulfate, aluminum sulfate/potassium, sodium pyrrolidone carboxylate, sodium lactate, potassium lactate, sodium ascorbate, sodium salicylate, sodium isethionate and the like. Preferred are sodium chloride, sodium pyrrolidone carboxylate and sodium lactate.

The content of component (C): electrolyte in the composition of the present invention is generally 0.01 - 20 wt%, preferably 0.05 - 5 wt%, relative to the total amount of the composition.

### 4. Component (D): alcohol

The "alcohol" in the present specification is preferably a straight-chain or branched-chain alcohol having 1 - 6 carbon atoms. Specific preferable examples of the "alcohol" include methanol, ethanol, propanol, isopropanol, butanol, isobutanol, sec-butanol, tert-butanol, pentanol, hexanol and the like, and more preferred are ethanol, propanol and isopropanol.

The content of component (D): alcohol in the composition of the present invention is generally 3 - 80 wt% relative to the total amount of the composition, and preferably 10 - 65 wt% relative to the total amount of the composition.

While pH of the composition of the present invention is not particularly limited, it is generally pH 3 - 9, preferably pH 4 - 7.5, from the aspect of the maintenance of the thickening property.

The present invention also relates to a cosmetic containing the aforementioned composition of the present invention.

Specific examples of the cosmetic in the present invention include facial cleanser, skin lotion, milky lotion, cream, gel, serum, facial mask, mask, soap, body shampoo, face powder, foundation, lip rouge, blush, eyeliner, mascara, eye shadow, eyebrow pencil, shampoo, rinse, conditioner, hair styling agent, hair treatment and the like.

The cosmetics of the present invention may contain components that can be generally added to a cosmetics, as long as the effect of the present invention is not inhibited. Specific examples thereof include oil, chelating agent, surfactant, powder, amino acids, polyvalent alcohol, polyamino acid and salt thereof, water-soluble polymer, sugar alcohol and alkylene oxide adduct thereof, lower alcohol, animal and plant extract, nucleic acid, vitamin, enzyme, anti-inflammatory agent, antimicrobial agent, preservative, antioxidant, ultraviolet absorber, adiaphoretic, pigment, dye, oxidation dye, organic and inorganic powder, pH adjuster, pearly sheen agent and wetting agent.

The present invention also relates to a method of producing a composition with improved viscosity stability, comprising a step of adding component (A): compound (1) as defined in claim 1 with z = 7 or 8, or a salt thereof to component (B): an anionic water-soluble polymer.

In the present specification, "with improved viscosity stability" means that the composition was improved to maintain viscosity in a wide pH range even in the presence of electrolyte or alcohol. The wide pH range refers to pH 4 - 8, and the viscosity of the composition of the present invention generally means a viscosity requested by the use or kind of the composition.

As component (A): compound (1) or a salt thereof, one produced by the method described in the present specification, as well as the methods described in patent document 2, non-patent document 2 and non-patent document 3 can be used.

As component (B): an anionic water-soluble polymer, a commercially available product can be used.

The present invention also relates to a method of suppressing syneresis or aggregation of a composition, comprising a step of adding component (B): an anionic water-soluble polymer to component (A): compound (1) or a salt thereof. Syneresis means separation of water from the composition, and aggregation means change from a uniform state to a non-uniform state. The above-mentioned addition method can be performed according to a conventional method. In addition, the amount to be added and specific components are as described above.

The present invention also relates to a method of stabilizing viscosity of a composition, comprising a step of adding component (A): compound (1) or a salt thereof to component (B): an anionic water-soluble polymer in the presence of electrolyte or alcohol. The addition method can be performed according to a conventional method. In addition, the amount to be added and specific components are as described above.

### [Examples]

The present invention is concretely explained in the following by referring to Examples. The present invention is not limited by the Examples. Unless particularly indicated, "%" means "wt%".

### Production Example 1: Synthesis of bis (N^{ε}-lauroyl-L-lysine)sebacoylamide disodium salt

N^{ε}-lauroyl-L-lysine (8.2 g, 25 mmol) was dissolved in water (70 g) and 25% aqueous sodium hydroxide solution (10 g), and diethyl ether (80 g) was added. Sebacoyl chloride (3.3 g, 14 mmol) was slowly added to the ether layer. The two-layer solution was stirred for about 1 hr while maintaining at 0°C, and then at room temperature for 23 hr. Then, 75% sulfuric acid was added dropwise to adjust to pH 2, the obtained white precipitate was collected by filtration, washed well with water and dried. The obtained compound was dissolved in an aqueous sodium hydroxide solution to give a 10% aqueous bis(N^{ε}-lauroyl-L-lysine)sebacoyl amide disodium salt solution.

### Production Example 2: Synthesis of bis(N^{ε}-lauroyl-L-lysine)sebacoylamide ditriethanolamine salt

N^{ε}-lauroyl-L-lysine (8.2 g, 25 mmol) was dissolved in water (70 g) and 25% aqueous sodium hydroxide solution (10 g), and diethyl ether (80 g) was added. Sebacoyl chloride (3.3 g, 14 mmol) was slowly added to the ether layer. The two-layer solution was stirred for about 1 hr while maintaining at 0°C, and then at room temperature for 23 hr. Then, 75% sulfuric acid was added dropwise to adjust to pH 2, the obtained white precipitate was collected by filtration, washed well with water and dried. The obtained compound was added to water, and the mixture was adjusted to pH 10 with triethanolamine to give a 10% aqueous bis(N^{ε}-lauroyl-L-lysine)sebacoyl amide ditriethanolamine salt solution.

### Production Example 3: Synthesis of bis(N^{ε}-octanoyl-L-lysine)sebacoylamide disodium salt

N^{ε}-octanoyl-L-lysine (6.8 g, 25 mmol) was dissolved in water (70 g) and 25% aqueous sodium hydroxide solution (10 g), and diethyl ether (80 g) was added. Sebacoyl chloride (3.3 g, 14 mmol) was slowly added to the ether layer. The two-layer solution was stirred for about 1 hr while maintaining at 0°C, and then at room temperature for 23 hr. Then, 75% sulfuric acid was added dropwise to adjust to pH 2, the obtained white precipitate was collected by filtration, washed well with water and dried. The obtained compound was dissolved in an aqueous sodium hydroxide solution to give a 10% aqueous bis(N^{ε}-octanoyl-L-lysine)sebacoyl amide disodium salt solution.
¹H-NMR of bis(N^{ε}-octanoyl-L-lysine)sebacoyl amide (free form)
¹H-NMR (400 MHz, DMSO-d₆, TMS, 25 °C) : δ 0.85 (t, J = 6.8 Hz, 6H), 1.20-1.29 (m, 28H), 1.32-1.38 (m, 4H), 1.45-1.50 (m, 8H), 1.54-1.59 (m, 4H), 2.02 (t, J = 7.4 Hz, 4H), 2.09 (t, J = 7.4 Hz, 4H), 2.99 (q, J = 6.5 Hz, 4H), 4.08-4.47 (m, 2H), 7.73 (t, J = 5.6 Hz, 2H), 7.97 (d, J = 8.0 Hz, 2H).

### Experimental Example 1

The syneresis and aggregation suppressive effect of the composition of the present invention was evaluated. The preparation of the composition, evaluation method and evaluation criteria are as follows.

### Preparation of composition of Example 1

Component (A) (compound synthesized in Production Example 1) was added to component (B) dispersed in water by stirring in advance, and the mixture was stirred at room temperature (wt% of each component is described in Table 1). The obtained mixture was divided into two, and the pH was adjusted to pH 4.8 and pH 5.5. The prepared composition was completely sealed tightly in a 30 mL glass bottle, and preserved at room temperature for not less than 5 days.

### Preparation of composition of Comparative Example 1

By a method similar to that for the preparation of the composition of Example 1 except that component (A) was not added, a composition was prepared, and preserved at room temperature for not less than 5 days.

### Evaluation 1: Syneresis (whether water is separated from composition)

The glass bottle containing the composition (pH 5.5) prepared as mentioned above was placed upside down, visually observed and evaluated according to the following criteria.
⊙: syneresis not confirmed, water is hardly separated in glass bottle placed upside down
×: syneresis confirmed, water is clearly separated in glass bottle placed upside down

### Evaluation 2: Aggregation suppressive effect

The composition (pH 4.8) prepared as mentioned above was visually observed and evaluated according to the following criteria.
⊙: aggregate not confirmed
×: aggregate clearly confirmed

The results are shown in Table 1.

**Table 1**

| | | Example 1 | Comparative Example 1 |
|---|---|---|---|
| component (A) | compound of Production Example 1 (as 10% aqueous solution) | 1.0 | 1.0 |
| component (B) | carboxyvinyl polymer | 0.2 | - |
| pH adjust | citric acid 3Na | 0.1 | 0.1 |
| | citric acid | q.s. | q.s. |
| | NaOH | q.s. | q.s. |
| | water | balance | balance |
| evaluation 1: | syneresis | ⊙ | × |
| evaluation 2: | aggregation suppressive effect | ⊙ | × |

In Comparative Example without addition of component (B), water was separated from the composition, and aggregation occurred (Comparative Example 1). On the other hand, water was not separated from the composition of the present invention added with component (B), and aggregation was suppressed (Example 1).

### Experimental Example 2

The viscosity and usability of the composition of the present invention at each pH in the presence of electrolyte or alcohol were evaluated. The preparation of the composition, evaluation method and evaluation criteria are as follows.

### Preparation of compositions of Examples 2 - 8

Component (A) (compound synthesized in Production Examples 1 and 3) was added to component (B) dispersed in water by stirring in advance, component (C) (electrolyte) (sodium pyrrolidone carboxylate (50% aqueous solution); AJIDEW NL-50 (manufactured by Ajinomoto Co., Inc.)) was added, and the mixture was stirred at room temperature (wt% of each component is described in Table 2). The obtained mixture was divided into four, and the pH was adjusted to pH 4.8, pH 5.5, pH 6.5 and pH 7.5. The prepared test compositions were filled and completely sealed tightly in a 30 mL glass bottle, and preserved at room temperature for 5 to 8 days.

### Preparation of compositions of Comparative Examples 2 - 8

By a method similar to that for the preparation of the compositions of Examples 2 - 8 except that component (A) was not added, each composition was prepared, and preserved at room temperature for 5 to 8 days.

### Preparation of compositions of Examples 9 - 18

Component (A) (compound synthesized in Production Example 2) was added to a mixture of component (B) dispersed in water by stirring in advance and component (D) (alcohol), and the mixture was stirred well at room temperature (wt% of each component is described in Table 3). The obtained mixture was divided into four, and the pH was adjusted to pH 4.8, pH 5.5, pH 6.5 and pH 7.5 by adding those recited in the item of pH adjust in the Table. The prepared test compositions were filled and completely sealed tightly in a 30 mL glass bottle, and preserved at room temperature for 5 to 8 days.

### Preparation of compositions of Comparative Examples 9 and 10

By a method similar to that for the preparation of the compositions of Examples 9 - 18 except that component (A) was not added, each composition was prepared, and preserved at room temperature for 5 to 8 days.

### Evaluation 3: Stability to electrolyte or alcohol

The glass bottle filled with the composition (pH 5.5) prepared and preserved as mentioned above was placed upside down, visually observed and evaluated according to the following criteria.
⊙: does not drip even from glass bottle placed upside down
×: drips from glass bottle when placed upside down

### Evaluation 4: pH stability

The glass bottles filled with respective compositions (pH 4.8, pH 5.5, pH 6.5, pH 7.5) prepared and preserved as mentioned above were placed upside down, visually observed and evaluated according to the following criteria.
⊙: does not drip even from glass bottle placed upside down at three or more pHs
○: does not drip even from glass bottle placed upside down at two pHs
×: does not drip even from glass bottle placed upside down at one or less pHs

### Evaluation 5: usability (stickiness)

The compositions (25 - 35 mg) prepared as mentioned above (pH 5.5) were applied to the skin within the range of 8×2 cm on the inner side of the forearm of the test subjects, and the presence of stickiness during or immediately after the application was examined, and evaluated according to the following criteria.
⊙: not more than one test subject acknowledges stickiness
○: not less than 2 and less than 4 test subjects acknowledge stickiness
Δ: not less than 4 and less than 6 test subjects acknowledge stickiness
×: not less than 6 test subjects acknowledge stickiness

### Evaluation 6: usability (sliminess)

The compositions (25 - 35 mg) prepared as mentioned above (pH 5.5) were applied to the skin within the range of 8×2 cm on the inner side of the forearm of the test subjects, and the presence of sliminess during or immediately after the application was examined, and evaluated according to the following criteria.
⊙: not more than one test subject acknowledges sliminess
○: not less than 2 and less than 4 test subjects acknowledge sliminess
Δ: not less than 4 and less than 6 test subjects acknowledge sliminess
×: not less than 6 test subjects acknowledge sliminess

The results are shown in Table 2 and Table 3.

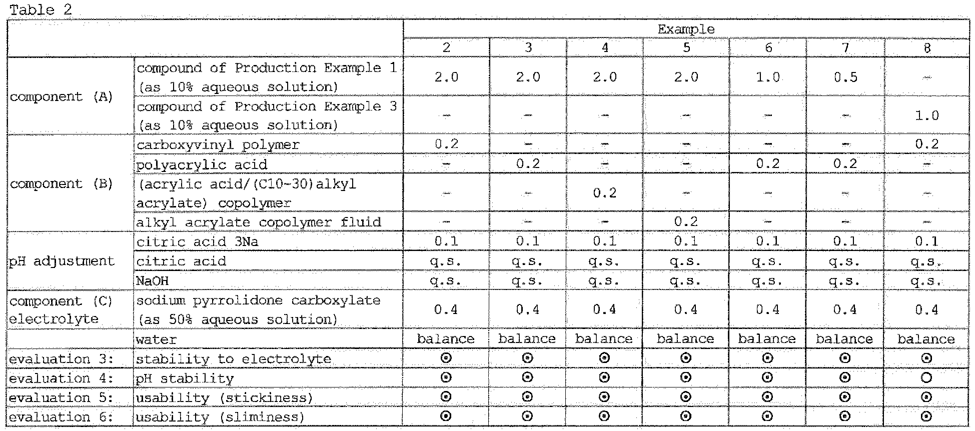

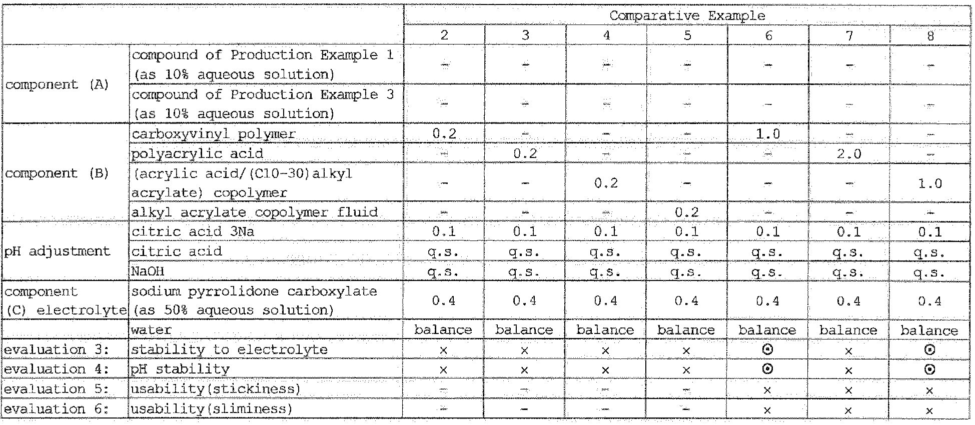

**Table 3**

| | | Example | | | | | | | | | | Comparative Example | |
|---|---|---|---|---|---|---|---|---|---|---|---|---|---|
| | | 9 | 10 | 11 | 12 | 13 | 14 | 15 | 16 | 17 | 18 | 9 | 10 |
| component (A) | compound of Production Example 2 (as 10% aqueous solution) | 2.0 | 2.0 | 2.0 | 2.0 | 2.0 | 2.0 | 0.5 | 5.0 | 10.0 | 1.0 | - | - |
| component (B) | carboxyvinyl polymer | 0.2 | 0.2 | 0.2 | 0.2 | - | 0.2 | 0.2 | 0.2 | 0.2 | 0.2 | 0.2 | 0.5 |
| | (acrylic acid/(C10-30)alkyl acrylate) copolymer | - | - | - | - | 0.2 | - | - | - | - | - | - | - |
| pH adjustment | citric acid 3Na aqueous solution (10%) | 0.1 | 0.1 | 0.1 | 0.1 | 0.1 | 0.1 | 0.1 | 0.1 | 0.1 | 0.1 | 0.1 | 0.1 |
| | citric acid | q.s. | q.s. | q.s. | q.s. | q.s. | q.s. | q.s. | q.s. | q.s. | q.s. | q.s. | q.s. |
| | triethanolamine | q.s. | q.s. | q.s. | q.s. | q.s. | q.s. | q.s. | q.s. | q.s. | q.s. | q.s. | q.s. |
| component (D) alcohol | ethanol | 10.0 | 25.0 | 50.0 | 65.0 | 35.0 | 25.0 | 25.0 | 25.0 | 65.0 | - | 65.0 | 65.0 |
| | isopropanol | - | - | - | - | - | - | - | - | - 25.0 | 25.0 | - | - |
| | water | balance | balance | balance | balance | balance | balance | balance | balance | balance | balance | balance | balance |
| evaluation 3: | stability to alcohol | ⊙ | ⊙ | ⊙ | ⊙ | ⊙ | ⊙ | ⊙ | ⊙ | ⊙ | ⊙ | × | × |
| evaluation 4: | pH stability | ⊙ | ⊙ | ○ | ○ | ⊙ | ⊙ | ⊙ | ⊙ | ○ | ⊙ | × | × |
| evaluatior 5: | usability (stickiness) | ⊙ | ⊙ | ⊙ | ⊙ | ⊙ | ⊙ | ⊙ | ⊙ | ⊙ | ⊙ | - | - |
| evaluatior 6: | usability (sliminess) | ⊙ | ⊙ | ⊙ | ⊙ | ⊙ | ⊙ | ⊙ | ⊙ | ⊙ | ⊙ | - | - |

Comparative Examples without addition of component (A) were inferior in the sense of use during application, since sufficient viscosity was not achieved when the amount of component (B) was suppressed to focus on the sense of use (Comparative Examples 2 - 5, 9 and 10), and stickiness and sliminess were produced when the amount of component (B) was increased to supplement viscosity (Comparative Examples 6 - 8). On the other hand, the compositions of the present invention added with component (A) could maintain thickening property in a wide pH range, and decreased stickiness and sliminess (Examples 2 - 18).

Preferable blending examples of the composition of the present invention are explained below.

### Blending Example 1 moisturizing gel

A moisturizing gel of the formulation shown in the following Table 4 was prepared according to a conventional method.

**[Table 4]**

| | weight % |
|---|---|
| butylene glycol | 5.00 |
| glycerol | 4.00 |
| polyquaternium-61 | 0.05 |
| sodium pyrrolidone carboxylate | 0.20 |
| hydrogenated lecithin, glycerol | 0.10 |
| pentasodium pentetate | 0.05 |
| PEG-40 hydrogenated castor oil | 0.20 |
| phytosteryl/behenyl/2-octyldodecyl lauroyl glutamate | 0.15 |
| tri(caprylic/capric acid)glyceryl | 0.05 |
| isostearyl neopentanoate | 0.05 |
| arnica flower extract, cucumber fruit extract, English ivy extract, sambucus nigra extract, mauritiana Malva extract, parietary extract, butylene glycol, water | 0.10 |
| compound of Production Example 1 (10%) | 2.00 |
| (acrylates/(C10-30)alkyl acrylate) crosspolymer | 0.40 |
| arginine | 0.40 |
| sodium decanoyl prolinate, water | 1.67 |
| PCA ethyl cocoy arginate | 0.10 |
| water | balance |
| total | 100.00 |

### Blending Example 2 hand sanitizer

A hand sanitizer of the formulation shown in the following Table 5 was prepared according to a conventional method.

**[Table 5]**

| | weight % |
|---|---|
| carboxyvinyl polymer | 0.50 |
| compound of Production Example 1 (10%) | 1.00 |
| ethanol | 70.00 |
| aminomethyl propanol | 0.32 |
| glycerol | 2.00 |
| sodium acetylhyaluronate | 0.01 |
| allantoin | 0.10 |
| water | balance |
| total | 100.00 |

### Blending Example 3 hair tonic

A hair tonic of the formulation shown in the following Table 6 was prepared according to a conventional method.

**[Table 6]**

| | weight % |
|---|---|
| water | balance |
| Beheness-30 | 0.50 |
| compound of Production Example 1 (10%) | 0.50 |
| (acrylates/(C10-30)alkyl acrylate) crosspolymer | 0.05 |
| ethanol | 30.00 |
| panthenol | 0.10 |
| camphor | 0.05 |
| menthol | 0.10 |
| total | 100.00 |

### Blending Example 4 spray sunscreen

A spray sunscreen of the formulation shown in the following Table 7 was prepared according to a conventional method.

**[Table 7]**

| | weight % |
|---|---|
| t-butyl methoxydibenzoylmethane | 5.0 |
| octocrylene | 4.5 |
| polysilicone-15 | 3.0 |
| homosalate | 10.0 |
| tocopheryl acetate | 1.5 |
| panthenol | 0.2 |
| isononyl isononanoate | 4.0 |
| (C12-15)alkyl benzoate | 15. 0 |
| Phenyl trimethicone | 2.0 |
| dicaprylyl carbonate | 13.5 |
| BHT | 0.1 |
| cyclohexasiloxane | 6.0 |
| diethylhexyl butamido triazone | 2.0 |
| (VP/hexadecene) copolymer | 1.0 |
| (acrylates/octylacrylamide) copolymer | 0.8 |
| compound of Production Example 1 (10%) | 1.0 |
| ethanol | balance |
| total | 100.0 |

### Blending Example 5 after-shave gel

An after-shave gel of the formulation shown in the following Table 8 was prepared according to a conventional method.

**[Table 8]**

| | weight % |
|---|---|
| (hydroxyethyl acrylate/acryloyldimethyltaurine) copolymer sodium | 1.00 |
| water | balance |
| potassium sorbate | 0.20 |
| Food Blue No.1 | 0.10 |
| Food Purple No.201 | 0.02 |
| glutamic acid diacetic acid tetra sodium salt | 0.20 |
| betaine, sodium pyrrolidone carboxylate, sorbitol, serine, glycine, glutamic acid, alanine, lysine, arginine, threonine, proline, methylparaben, propylparaben, water | 3.00 |
| calcium pantothenate, niacinamide, sodium ascorbyl phosphate, tocopheryl acetate, pyridoxine hydrochloride, maltodextrin, sodium starch octenylsuccinate, silica | 0.14 |
| Butylene glycol, water, Rome camomile flower extract, Calendula officinalis flower extract, Centaurea cyanus flower extract, camomile flower extract, St. John's wort flower/leaf/stem extract, tilia cordata flower extract | 0.30 |
| PEG-11 methyl ether dimethicone | 5.00 |
| ethanol | 3.00 |
| glycerol | 3.00 |
| water | 10.00 |
| compound of Production Example 1 (10%) | 1.00 |
| sodium hydroxide | 0.20 |
| flavor: ethanol, limonene, citral, octanal, benzyl alcohol, lemon peel oil, orange peel oil, mandarin orangeleaf oil | 0.20 |
| citric acid | 0.21 |
| total | 100.00 |

The cosmetics of Blending Examples 1 - 5 showed sufficient viscosity, and suppressed stickiness and sliminess.

The details of the materials used are as follows.
butylene glycol: 1,3-butylene glycol (manufactured by Daicel Corporation)
glycerol: concentrated glycerol for cosmetics (manufactured by Sakamoto kogyo Co., Ltd.)
polyquaternium-61: lipodure-S (manufactured by NOF CORPORATION) sodium pyrrolidone carboxylate: AJIDEW NL-50 (manufactured by Ajinomoto Co., Inc.)
hydrogenated lecithin, glycerol: Lecinol SH50 (manufactured by Nikko Chemicals)
pentasodium pentetate: Chelest P-SD (manufactured by Chubu Chelest)
PEG-40 hydrogenated castor oil: EMALEX HC-40 (manufactured by Nihon Emulsion Co., Ltd.)
phytosteryl/behenyl/2-octyldodecyl lauroyl glutamate: ELDEW PS-304R (manufactured by Ajinomoto Co., Inc.)
tri(caprylic/capric acid)glyceryl: EMALEX KTG (manufactured by Nihon Emulsion Co., Ltd.)
isostearyl neopentanoate: neolite 180P (manufactured by Kokyu Alcohol Kogyo Co., Ltd.)
arnica flower extract, cucumber fruit extract, English ivy extract, sambucus nigra extract, mauritiana Malva extract, parietary extract, butylene glycol, water: PHYTELENE EGX-251 (manufactured by GREENTECH S.A.)
(acrylates/(C10-30)alkyl acrylate) crosspolymer: carbopol Ultrez 20 (manufactured by Lubrizol Advanced Materials)
sodium decanoyl prolinate, water: PRODEW P-DS-12 (manufactured by Ajinomoto Co., Inc.)
PCA ethyl cocoy arginate: CAE (manufactured by Ajinomoto Co., Inc.)
carboxyvinyl polymer: CARBOPOL 940 POLYMER (manufactured by Lubrizol Advanced Materials)
aminomethyl propanol: AMP-ULTRA PC 1000 (manufactured by DOW) sodium acetylhyaluronate: (manufactured by Shiseido Co., Ltd.)
allantoin: (manufactured by KAWAKEN fine chemicals co., Ltd.)
beheness-30: NIKKOL BB-30 (manufactured by Nikko Chemicals)
panthenol: D-panthenol USP (manufactured by BASF)
camphor: the Japanese Pharmacopoeia dl-camphor (manufactured by Nippon Fine Chemical)
menthol: menthol (manufactured by Takasago International Corporation)
t-butyl methoxydibenzoylmethane: Parsol 1789 (manufactured by DSM NUTRITIONAL)
octocrylene: Parsol 340 (manufactured by DSM NUTRITIONAL)
polysilicone-15: Parsol SLX (manufactured by DSM NUTRITIONAL)
homosalate: Parsol HMS (manufactured by DSM NUTRITIONAL)
isononyl isononanoate: SALACOS 99 (manufactured by Nisshin OilliO)
(C12-15)alkyl benzoate: FINSOLV TN (manufactured by INNOSPEC)
phenyltrimethicone: EMALEX SO-29 (manufactured by Nihon Emulsion Co., Ltd.)
dicaprylyl carbonate: CETIOL CC (manufactured by BASF)
cyclohexasiloxane: DC246 Fluid (manufactured by Toray-Dow Corning Corporation)
diethylhexyl butamido triazone: UVASORB HEB (manufactured by 3V Sig)
(VP/hexadecene) copolymer: Antaron V216 (manufactured by ISP)
(acrylates/octylacrylamide) copolymer: DERMACRYL 79 (manufactured by AKZO NOBEL)
(hydroxyethyl acrylate/acryloyldimethyltaurine) copolymer sodium: SEPIMAX C (manufactured by SEPPIC)
Food Blue No. 2: (manufactured by Kishi Kasei Co., Ltd.)
Food Purple No. 201: (manufactured by Kishi Kasei Co., Ltd.)
glutamic acid diacetic acid tetra sodium salt: DISSOLVINE GL-47-S (manufactured by AKZO NOBEL)
betaine, sodium pyrrolidone carboxylate, sorbitol, serine, glycine, glutamic acid, alanine, lysine, arginine, threonine, proline, methylparaben, propylparaben, water: PRODEW 400 (manufactured by Ajinomoto Co., Inc.)
calcium pantothenate, niacinamide, sodium ascorbyl phosphate, tocopheryl acetate, pyridoxine hydrochloride, maltodextrin, sodium starch octenylsuccinate, silica: BeauPlex VH (manufactured by DSM NUTRITIONAL)
butylene glycol, water, Rome camomile flower extract, Calendula officinalis flower extract, Centaurea cyanus flower extract, camomile flower extract, St. John's wort flower/leaf/stem extract, TILIA CORDATA FLOWER extract: Pharcorex BX44 (manufactured by ICHIMARU PHARCOS Co., Ltd.)
PEG-11 methyl ether dimethicone: KF-6011 (manufactured by Shin-Etsu Chemical Co., Ltd.)

### [Industrial Applicability]

The present invention can provide a composition which suppresses syneresis and aggregation over time, maintains desired viscosity in a wide pH range even in the presence of electrolyte or alcohol, and suppresses stickiness and sliminess.

## Claims

1. A composition comprising component (A): a compound represented by the formula (1) 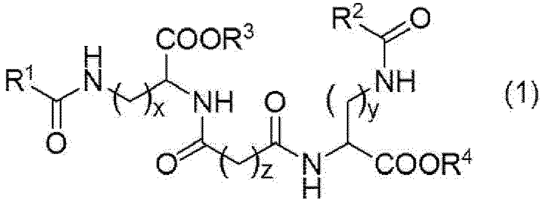 wherein
R¹ and R² are each independently an alkyl group having 5 - 21 carbon atoms or an alkenyl group having 5 - 21 carbon atoms,
R³ and R⁴ are each independently a hydrogen atom, an alkyl group having 1 - 22 carbon atoms or an alkenyl group having 2 - 22 carbon atoms,
z is 7 or 8,
x and y are each independently an integer of 2 - 4, or a salt thereof, and
component (B): an anionic water-soluble polymer.

2. The composition according to claim 1, wherein component (A) is a compound of the aforementioned formula (1) wherein x and y are each 4, or a salt thereof.

3. The composition according to claim 1 or 2, wherein component (A) is a compound of the aforementioned formula (1) wherein R¹ and R² are each independently a straight-chain alkyl group having 5 - 15 carbon atoms, or a salt thereof.

4. The composition according to any one of claims 1 to 3, wherein component (A) is a compound of the aforementioned formula (1) wherein R³ and R⁴ are each a hydrogen atom, or a salt thereof.

5. The composition according to any one of claims 1 to 4, wherein component (A) is a compound of the aforementioned formula (1) wherein R¹ and R² are each a straight-chain alkyl group having 5 - 15 carbon atoms, R³ and R⁴ are each a hydrogen atom, and x and y are each 4, or a salt thereof.

6. The composition according to any one of claims 1 to 5, wherein component (A) is a compound selected from bis (N^{ε}-lauroyl-L-lysine)sebacoyl amide and bis(N^{ε}-octanoyl-L-lysine)sebacoyl amide, or a salt thereof.

7. The composition according to any one of claims 1 to 6, wherein component (B) is an anionic water-soluble polymer having a carboxyl group.

8. The composition according to any one of claims 1 to 6, wherein component (B) is at least one kind of anionic water-soluble polymer selected from the group consisting of a carboxyvinyl polymer, polyacrylic acid or a salt thereof, crosslinked polyacrylic acid or a salt thereof, (acrylic acid/(C10-30)alkyl acrylate) copolymer, alkyl acrylate copolymer fluid, carboxymethylcellulose or a salt thereof, xanthan gum, and alginic acid or a salt thereof.

9. The composition according to any one of claims 1 to 8, further comprising component (C): electrolyte or component (D) :alcohol.

10. The composition according to claim 9, wherein component (C) is at least one kind of electrolyte selected from the group consisting of sodium chloride, sodium pyrrolidone carboxylate and sodium lactate.

11. The composition according to claim 9, wherein component (D) is at least one kind of alcohol selected from the group consisting of ethanol, propanol and isopropanol.

12. The composition according to claim 9, wherein:
the content of component (A) is 0.001 - 10 wt%;
the content of component (B) is 0.05 - 10 wt%;
the content of component (C), when present, is 0.01 - 20 wt%; or
the content of component (D), when present, is 3 - 80 wt%; and/or
the pH is 3- 9;
the weight precentages being relative to the total amount of the composition.

13. A cosmetic comprising the composition according to any one of claims 1 to 12.

14. A method of producing a composition with improved viscosity stability, comprising a step of blending component (A): a compound represented by the formula (1) 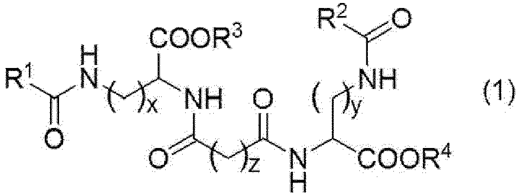 wherein
R¹ and R² are each independently an alkyl group having 5 - 21 carbon atoms or an alkenyl group having 5 - 21 carbon atoms,
R³ and R⁴ are each independently a hydrogen atom, an alkyl group having 1 - 22 carbon atoms or an alkenyl group having 2 - 22 carbon atoms,
z is 7 or 8,
x and y are each independently an integer of 2 - 4, or a salt thereof, and
component (B): an anionic water-soluble polymer.

## Patentansprüche

1. Zusammensetzung, umfassend Komponente (A): eine Verbindung der Formel (1) 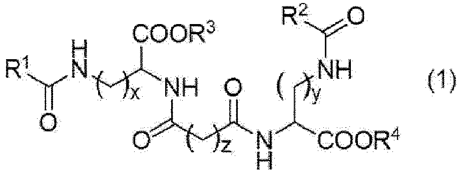 wobei
R¹ und R² jeweils unabhängig voneinander eine Alkylgruppe mit 5 - 21 Kohlenstoffatome oder eine Alkenylgruppe mit 5 - 21 Kohlenstoffatomen sind,
R³ und R⁴ jeweils unabhängig voneinander ein Wasserstoffatom, eine Alkylgruppe mit 1 - 22 Kohlenstoffatomen oder eine Alkenylgruppe mit 2 - 22 Kohlenstoffatomen sind,
z 7 oder 8 ist,
x und y jeweils unabhängig voneinander eine ganze Zahl von 2 - 4 sind, oder ein Salz davon und
Komponente (B): ein anionisches wasserlösliches Polymer.

2. Zusammensetzung nach Anspruch 1, wobei die Komponente (A) eine Verbindung der vorgenannten Formel (1) ist, worin x und y jeweils 4 sind, oder ein Salz davon.

3. Zusammensetzung nach Anspruch 1 oder 2, wobei die Komponente (A) eine Verbindung der vorstehend genannten Formel (1) ist, worin R¹ und R² jeweils unabhängig voneinander eine geradkettige Alkylgruppe mit 5 - 15 Kohlenstoffatomen sind, oder ein Salz davon.

4. Zusammensetzung nach einem der Ansprüche 1 bis 3, wobei die Komponente (A) eine Verbindung der vorgenannten Formel (1) ist, worin R³ und R⁴ jeweils ein Wasserstoffatom ist, oder ein Salz davon.

5. Zusammensetzung nach einem der Ansprüche 1 bis 4, wobei die Komponente (A) eine Verbindung der vorgenannten Formel (1) ist, worin R¹ und R² jeweils eine geradkettige Alkylgruppe mit 5 - 15 Kohlenstoffatomen sind, R³ und R⁴ jeweils ein Wasserstoffatom sind und x und y jeweils 4 sind, oder ein Salz davon.

6. Zusammensetzung nach einem der Ansprüche 1 bis 5, wobei die Komponente (A) eine Verbindung ist, die ausgewählt ist aus Bis(N^{ε}-)lauroyl-L-lysin)sebacoylamid und Bis(N^{ε}-octanoyl-L) lysin)sebacoylamid, oder ein Salz davon.

7. Zusammensetzung nach einem der Ansprüche 1 bis 6, wobei die Komponente (B) ein anionisches wasserlösliches Polymer mit einer Carboxylgruppe ist.

8. Zusammensetzung nach einem der Ansprüche 1 bis 6, wobei die Komponente (B) mindestens eine Art eines anionischen wasserlöslichen Polymers ist, ausgewählt aus der Gruppe bestehend aus einem Carboxyvinylpolymer, Polyacrylsäure oder einem Salz davon, vernetzter Polyacrylsäure oder einem Salz davon, (Acrylsäure/(C10-30)Alkylacrylat)-Copolymer, Alkylacrylat-Copolymerflüssigkeit, Carboxymethylcellulose oder einem Salz davon, Xanthangummi und Alginsäure oder einem Salz davon.

9. Die Zusammensetzung nach einem der Ansprüche 1 bis 8, ferner umfassend die Komponente (C): Elektrolyt oder die Komponente (D): Alkohol.

10. Zusammensetzung nach Anspruch 9, wobei die Komponente (C) mindestens eine Art eines Elektrolyts ist, ausgewählt aus der Gruppe bestehend aus Natriumchlorid, Natriumpyrrolidoncarboxylat und Natriumlactat.

11. Die Zusammensetzung nach Anspruch 9, wobei die Komponente (D) mindestens eine Art von Alkohol ist, ausgewählt aus der Gruppe bestehend aus Ethanol, Propanol und Isopropanol.

12. Die Zusammensetzung nach Anspruch 9, wobei:
der Gehalt der Komponente (A) 0,001 - 10 Gew.-% ist;
der Gehalt der Komponente (B) 0,05 - 10 Gew.-% ist;
der Gehalt der Komponente (C), falls vorhanden, 0,01 - 20 Gew.-% ist; oder
der Gehalt der Komponente (D), falls vorhanden, 3 - 80 Gew.-% ist; und/oder
der pH-Wert 3- 9 ist;
wobei die Gewichtsprozente sich auf die Gesamtmenge der Zusammensetzung beziehen.

13. Kosmetikum, umfassend die Zusammensetzung nach einem der Ansprüche 1 bis 12.

14. Verfahren zur Herstellung einer Zusammensetzung mit verbesserter Viskositätsstabilität, umfassend einen Schritt des Mischens der Komponente (A): eine Verbindung der Formel (1) 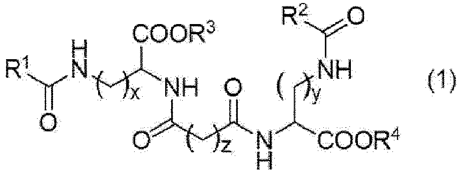 wobei
R¹ und R² jeweils unabhängig voneinander eine Alkylgruppe mit 5 - 21 Kohlenstoffatome oder eine Alkenylgruppe mit 5 - 21 Kohlenstoffatomen sind,
R³ und R⁴ jeweils unabhängig voneinander ein Wasserstoffatom, eine Alkylgruppe mit 1 - 22 Kohlenstoffatomen oder eine Alkenylgruppe mit 2 - 22 Kohlenstoffatomen sind,
z 7 oder 8 ist,
x und y jeweils unabhängig voneinander eine ganze Zahl von 2 - 4 sind, oder ein Salz davon und
der Komponente (B): ein anionisches wasserlösliches Polymer.

## Revendications

1. Composition comprenant un composant (A) : un composé représenté par la formule (1) 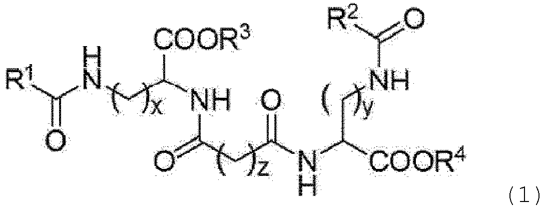 dans laquelle
chacun de R¹ et R² est indépendamment un groupe alkyle ayant 5 à 21 atomes de carbone ou un groupe alcényle ayant 5 à 21 atomes de carbone,
chacun de R³ et R⁴ est indépendamment un atome d'hydrogène, un groupe alkyle ayant 1 à 22 atomes de carbone ou un groupe alcényle ayant 2 à 22 atomes de carbone,
z est 7 ou 8,
chacun de x et y est indépendamment un entier de 2 à 4, ou un sel de celui-ci, et
un composant (B) : un polymère anionique soluble dans l'eau.

2. Composition selon la revendication 1, dans laquelle le composant (A) est un composé de formule (1) susmentionnée dans laquelle chacun de x et y vaut 4, ou un sel de celui-ci.

3. Composition selon la revendication 1 ou 2, dans laquelle le composant (A) est un composé de formule (1) susmentionnée dans laquelle chacun de R¹ et R² est indépendamment un groupe alkyle à chaîne linéaire ayant 5 à 15 atomes de carbone, ou un sel de celui-ci.

4. Composition selon l'une quelconque des revendications 1 à 3, dans laquelle le composant (A) est un composé de formule (1) susmentionnée dans laquelle chacun de R³ et R⁴ est un atome d'hydrogène, ou un sel de celui-ci.

5. Composition selon l'une quelconque des revendications 1 à 4, dans laquelle le composant (A) est un composé de formule (1) susmentionnée dans laquelle chacun de R¹ et R² est indépendamment un groupe alkyle à chaîne linéaire ayant 5 à 15 atomes de carbone, chacun de R³ et R⁴ est un atome d'hydrogène, et chacun de x et y vaut 4, ou un sel de celui-ci.

6. Composition selon l'une quelconque des revendications 1 à 5, dans laquelle le composant (A) est choisi parmi le bis(N^{ε}-lauroyl-L-lysine)sébaçoyle amide, et le bis (N^{ε}-octanoyl-L-lysine) sébaçoyle amide, ou un sel de celui-ci.

7. Composition selon l'une quelconque des revendications 1 à 6, dans laquelle le composant (B) est un polymère anionique soluble dans l'eau ayant un groupe carboxyle.

8. Composition selon l'une quelconque des revendications 1 à 6, dans laquelle le composant (B) est au moins un type de polymère anionique soluble dans l'eau choisi dans le groupe constitué par un polymère carboxyvinylique, un poly(acide acrylique) ou un sel de celui-ci, un poly(acide acrylique) réticulé ou un sel de celui-ci, un copolymère d'acide acrylique/acrylate d'alkyle en C₁₀ à C₃₀, un fluide copolymère d'acrylate d'alkyle, la carboxyméthylcellulose ou un sel de celle-ci, la gomme xanthane, et l'acide alginique ou un sel de celui-ci.

9. Composition selon l'une quelconque des revendications 1 à 8, comprenant en outre un composant (C) : un électrolyte, ou un composant (D) : un alcool.

10. Composition selon la revendication 9, dans laquelle le composant (C) est au moins un type d'électrolyte choisi dans le groupe constitué par le chlorure de sodium, le carboxylate de pyrrolidone sodique et le lactate de sodium.

11. Composition selon la revendication 9, dans laquelle le composant (D) est au moins un type d'alcool choisi dans l'ensemble constitué par l'éthanol, le propanol et l'isopropanol.

12. Composition selon la revendication 9, dans laquelle :
la teneur en composant (A) est de 0,001 à 10 % en poids ;
la teneur en composant (B) est de 0,05 à 10 % en poids ;
la teneur en =composant (C), quand il est présent, est de 0,01 à 20 % en poids ; ou
la teneur en composant (D), quand il est présent, est de 3 à 80 % en poids ; et/ou
le pH est de 3 à 9 ;
les pourcentages en poids étant par rapport à la quantité totale de la composition.

13. Cosmétique comprenant la composition selon l'une quelconque des revendications 1 à 12.

14. Procédé de production d'une composition ayant une meilleure stabilité de la viscosité, comprenant une étape de mélange d'un composant (A) :
un composé représenté par la formule (1) 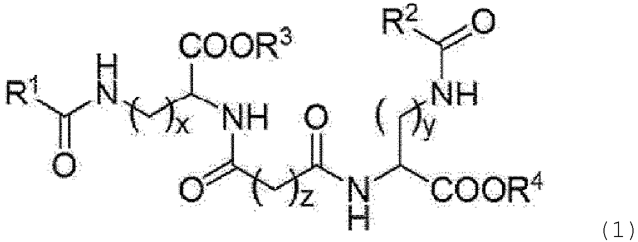 dans laquelle
chacun de R¹ et R² est indépendamment un groupe alkyle ayant 5 à 21 atomes de carbone ou un groupe alcényle ayant 5 à 21 atomes de carbone,
chacun de R³ et R⁴ est indépendamment un atome d'hydrogène, un groupe alkyle ayant 1 à 22 atomes de carbone ou un groupe alcényle ayant 2 à 22 atomes de carbone,
z est 7 ou 8,
chacun de x et y est indépendamment un entier de 2 à 4, ou un sel de celui-ci, et
d'un composant (B) : un polymère anionique soluble dans l'eau.
